(19) Europäisches Patentamt
European Patent Office
Office européen des brevets

(11) Numéro de publication: **0 211 745 B1**

(12) # FASCICULE DE BREVET EUROPEEN

(45) Date de publication du fascicule du brevet: **08.11.89**

(51) Int. Cl.[4]: **C 07 C 103/76**, A 61 K 31/16

(21) Numéro de dépôt: **86401643.1**

(22) Date de dépôt: **23.07.86**

(54) **Dérivé d'acide acétohydroxamique, procédé de préparation et utilisation en thérapeutique.**

(30) Priorité: **31.07.85 FR 8511685**

(43) Date de publication de la demande: **25.02.87 Bulletin 87/9**

(45) Mention de la délivrance du brevet: **08.11.89 Bulletin 89/45**

(84) Etats contractants désignés: **AT BE CH DE FR GB IT LI LU NL SE**

(56) Documents cités: **FR-A- 2 345 430**

(73) Titulaire: **LABORATOIRE L. LAFON Société anonyme dite:, 1 rue Georges Médéric, F-94701 Maisons-Alfort (FR)**

(72) Inventeur: **Lafon, Louis, 5, rue de l'Alboni, F-75016 Paris (FR)**

(74) Mandataire: **Clisci, Serge et al, S.A. FEDIT-LORIOT CONSEILS EN PROPRIETE INDUSTRIELLE 38, avenue Hoche, F-75008 Paris (FR)**

EP 0 211 745 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un dérivé d'acide acétohydroxamique en tant que produit industriel nouveau, à savoir l'acide 2-(5-chloro-4-nitro-2--méthoxybenzamido)-acétohydroxamique. Elle concerne également son procédé de préparation et son utilisation en thérapeutique, notamment en tant qu'agent sédatif.

On connaît de BE-A-852 738 le chlorhydrate de l'acide 2-(4-aminobenzamido)-acétohydroxamique qui a pour No. de Code : CRL 40 473 et qui a des effets sur le système nerveaux central (voir exemple 18 dudit brevet belge). On connaît également le chlorhydrate de l'acide 3-(3-aminobenzamido)-propionhydroxamique qui a pour No. de Code : CRL 40 816 et qui présente des propiétés antidépressives particulièrement intéressantes.

On vient de trouver de façon surprenante que le nouveau composé selon l'invention, à savoir l'acide 2-(5-chloro-4-nitro-2-méthoxybenzamido)-acétohydroxamique (No. de Code : CRL 40 636), qui est structurellement différent des produits connus précités, est particulièrement intéressant en thérapeutique eu égard à ses effets sur le système nerveux central (SNC).

En bref, le CRL 40 636 selon l'invention est remarquable par ses effets sédatifs, alors que (i) le CRL 40 473 présente des effets antidépresseurs et sédatifs modérés, et que (ii) le CRL 40 816 est plus antidépresseur que sédatif.

Les résultats d'essais comparatifs consignés dans le tableau I ci-après mettent en évidence que le CRL 40 636 (produit de l'exemple 1 selon l'invention) est plus actif et agit à des doses plus faibles que le CRL 40 473 (produit de comparaison CP-1) et le CRL 40 816 (produit de comparaison CP-2) précités, selon le test dit de l'agressivité intergroupes.

Selon l'invention on préconise une composition thérapeutique qui est caractérisée en ce qu'elle renferme, en association avec un excipient physiologiquement acceptable, l'acide 2-(5-chloro-4-nitro-2--méthoxybenzamido)-acétohydroxamique qui a pour formule développée:

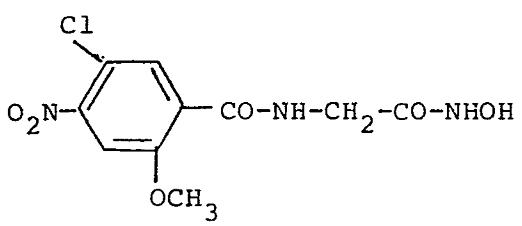

(I)

Le composé selon l'invention peut être préparé suivant une méthode connue en soi par application de mécanismes réactionnels classiques. Le procédé que l'on préconise ici consiste à faire réagir l'hydroxylamine avec un 5-chloro-4-nitro-2-méthoxybenzamidoacétate d'alkyle inférieur en $C_1$-$C_3$, à la température ambiante (15-20°C), pendant au moins 1 h, de préférence pendant au moins 4 h, à raison de 1 à 1,1 mole de $NH_2OH$ pour une mole de 5-chloro-4-nitro--2-méthoxybenzamidoacétate d'alkyle.

La synthèse totale du produit de l'invention, à partir de la 5-nitro-4-chloro-2-méthylaniline, est illustrée par le diagramme A qui suit.

DIAGRAMME A

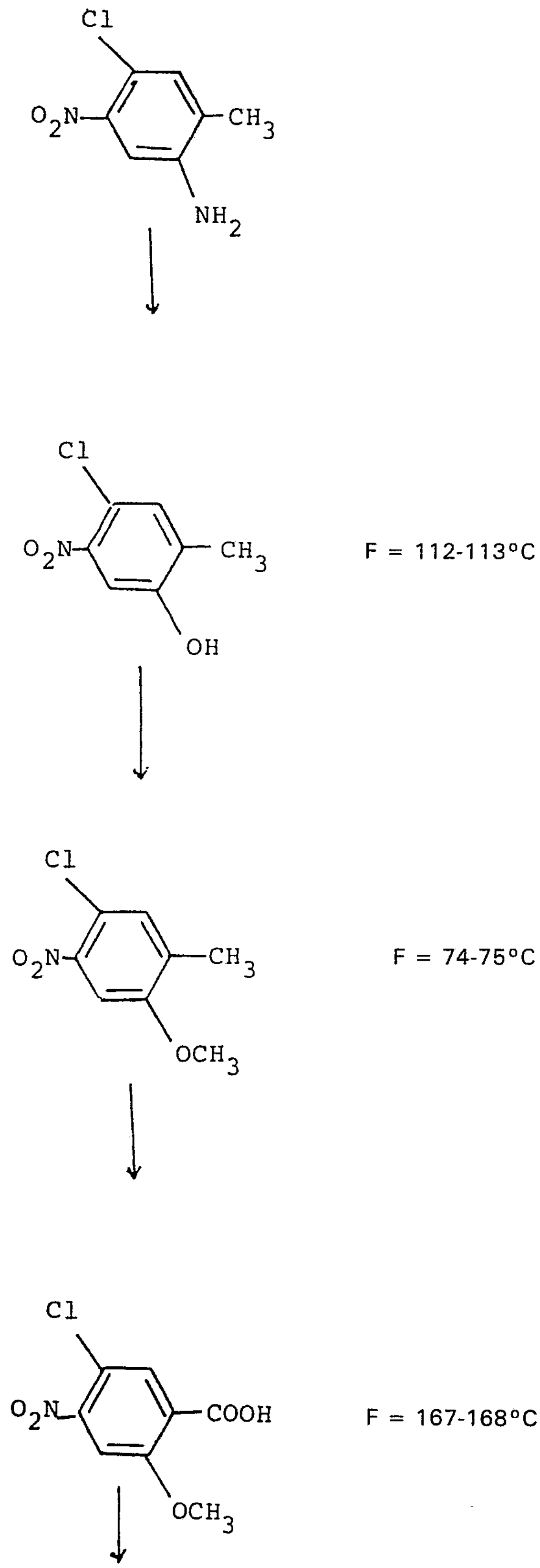

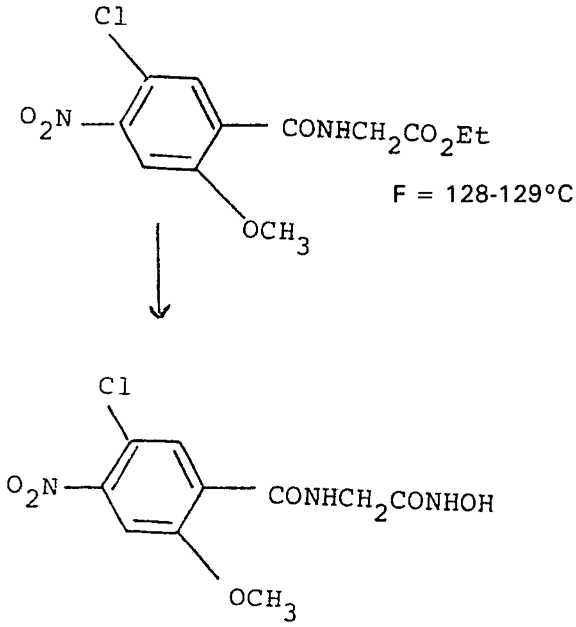


D'autres avantages et caractéristiques de l'invention seront mieux compris à la lecture qui va suivre d'un exemple de préparation, d'une part, et de résultats d'essais neuropsychopharmacologiques, d'autre part. Bien entendu, l'ensemble de ces éléments n'est nullement limitatif mais est donné à titre d'illustration.

*Exemple 1*

*Préparation de l'acide 2-(5-chloro-4-nitro-2-méthoxybenzamido)-acétohydroxamique.*
(No. de Code : CRL 40 636).

On prépare une solution d'hydroxylamine avec 5,6 g (0,08 mole) de chlorhydrate d'hydroxylamine et 3,68 g (0,16 Atg) de sodium dans 250 ml de méthanol. On ajoute 23,8 g (0,075 mole) de 5-chloro-4--nitro-2-méthoxybenzamidoacétate d'éthyle, et laisse une nuit en contact. On essore le sel de sodium, lave au méthanol et sèche. On reprend le produit séché ainsi obtenu dans 200 ml d'eau, filtre et précipite le filtrat avec HCl 3N. On essore, lave à l'eau, sèche. Par recristallisation du mélange pondéral $C_2H_5OH$-$H_2O$ (96:4), on obtient le CRL 40 636 avec un rendement de 44%. F = 196-198°C (avec décomposition).

On a résumé ci-après les résultats des essais qui ont été entrepris avec le composé selon l'invention. Dans la présente étude neuropsychopharmacologique le CRL 40 636 en suspension dans une solution aqueuse de gomme arabique a été administré par voie intrapéritonéale sous un volume de 20 ml/kg chez la souris mâle et de 5 ml/kg chez le rat mâle.

*I. TOXICITE*

Le CRL 40 636 n'est pas toxique. En effet la DL-O (dose maximale non mortelle) chez la souris par voie intrapéritonéale est supérieure à 1024 mg/kg.

*II. COMPORTEMENT GLOBAL ET REACTIVITES*

Des lots de trois animaux sont observés avant, puis 0,25 h, 0,50 h, 1 h, 2 h, 3 h, et 24 h après l'administration de CRL 40 636. On constate:

*1°) Chez la souris*

*à la dose de 512 mg/kg:*
— une sédation avec diminution des réactions de peur et d'agressivité, de la force musculaire et de la réactivité au toucher pendant 3 h,
— une hypothermie (−6,8°C) pendant plus de 3 h,
— une respiration déprimée pendant 2 à 3 h;

*à la dose de 128 mg/kg:*
— une sédation avec diminution de la réaction de peur et d'agressivité pendant 3 h,
— une hypothermie (−3,1°C) pendant 2 h,
— une respiration déprimée pendant 2 à 3 h;

*à la dose de 32 mg/kg:*
— une sédation (2 animaux sur 3) pendant 3 h,
— une hypothermie modérée (−2,2°C) pendant 1 h,
— une respiration déprimée (2 animaux sur 3) pendant 2 h;

*à la dose de 8 mg/kg:*
— une sédation inconstante (2 animaux sur 3) pendant 0,5 à 1 h; et,

*2°) Chez le rat*

*à la dose de 256 mg/kg:*
— une sédation pendant 24 h avec diminution de la force et du tonus musculaire pendant 1 à 3 h,
— une mydriase pendant 2 h,
— une respiration déprimée durant 1 à 3 h;

*à la dose de 64 mg/kg:*
— une sédation pendant 1 à 3 h avec diminution de la force et du tonus musculaires,
— une respiration déprimée durant 1 h;

*à la dose de 16 mg/kg:*
— une sédation (0,25 à 1 h) avec diminution de la force et du tonus musculaires,
— une respiration déprimée durant 0,25 à 1 h;

*à la dose de 4 mg/kg:*
— une sédation pendant 0,25 à 0,50 h accompagnée d'hypotonie musculaire,
— une respiration déprimée pendant 0,25 h.

*III. INTERACTION AVEC L'APOMORPHINE*

*1°) Chez la souris*

Une demi-heure après l'administration de CRL 40 636, des lots de 6 sours reçoivent par voie sous-cutanée une injection d'apomorphine à la dose de 1 ou 16 mg/kg. On constate que, aux doses de 128 et 512 mg/kg, le CRL 40 636 (qui administré seul exerce un effet hypothermisant important) ne s'oppose pas à l'hypothermie, à l'attitude de verticalisation ni aux stéréotypies induites par l'apomorphine chez la souris.

*2°) Chez le rat*

L'apomorphine est injectée par voie sous-cutanée

à la dose de 0,5 mg/kg à des lots de 6 rats 0,5 h après l'administration du CRL 40 636. On observe que les stéréotypies induites par l'apomorphine chez le rat ne sont pas modifiées par le CRL 40 636.

*IV.INTERACTION AVEC L'AMPHETAMINE*

0,5 h ou 1 h après l'administration de CRL 40 636, des lots de 6 rats reçoivent une injection par voie intrapéritonéale de 2 mg/kg d'amphétamine.

On constate que, dès la dose de 4 mg/kg, le CRL 40 636 antagonise fortement les stéréotypies amphétaminiques (alors que le CRL 40 816 précité n'entraîne qu'une diminution modérée desdites stéréotypies qu'à partir de la dose de 256 mg/kg i.p.); on observe en outre que, si l'antagonisme de ces stéréotypies par le CRL 40 636 est très intense au départ, il semble disparaître rapidement.

*V. INTERACTION AVEC LA RESERPINE*

Quatre heures après l'administration de réserpine (2,5 mg/kg), des lots de 6 souris reçoivent le CRL 40 636. On constate que le CRL 40 636 ne modifie pas l'hypothermie ni le ptôsis réserpiniques.

*VI. INTERACTION AVEC L'OXOTREMORINE*

L'oxotrémorine (0,5 mg/kg) est injectée par voie intrapéritonéale à des lots de 6 souris une demi-heure après l'administration de CRL 40 636. On observe que le CRL 40 636 qui exerce un effet hypothermisant important aux doses de 32, 128 et 512 mg/kg, ne s'oppose pas à ces doses à l'hypothermie induite par l'oxotrémorine, qu'il est dépourvu d'effet vis-à-vis des tremblements provoqués par l'oxotrémorine, mais qu'il semble entraîner une prolongation de la lacrymation (ce qui implique une action sur les symptômes cholinergiques périphériques).

*VII. ACTION SUR LE TEST DES QUATRE PLAQUES, LA TRACTION ET L'ELECTROCHOC*

Des lots de 10 souris sont soumis au test une demi-heure après l'administration de CRL 40 636. On constate que le CRL 40 636 ne provoque pas d'augmentation du nombre de passages punis; que, à forte dose, il entraîne chez 30% des animaux une incapacité motrice; et qu'il ne modifie pas les effets convulsivants et létaux de l'électrochoc.

*VIII. ACTION SUR LA MOTILITE SPONTANEE*

Une demi-heure après avoir reçu le CRL 40 636, les souris (6 par dose, 12 témoins) sont placées en actimètre où leur motilité est enregistrée pendant 0,5 h. On constate que, aux doses de 128 et 512 mg/kg, le CRL 40 636 entraîne une diminution importante de la motilité spontanée, alors que le CRL 40 816 précité ne provoque qu'une diminution modérée de ladite motilité à la dose de 512 mg/kg.

*IX. ACTION SUR L'AGRESSIVITE INTERGROUPES (étude comparative)*

Après avoir séjourné durant 3 semaines de part et d'autre d'une cloison opaque séparant leur cage par le milieu, des groupes de 3 souris mâles (chaque souris pesant environ 20 g) reçoivent les produits à tester, à savoir le CRL 40 636 (exemple 1), le CRL 40 473 (CP-1) et le CRL 40 816 (CP-2), par voie intrapéritonéale dans une solution aqueuse de gomme arabique, à raison de trois cages par produit et par dose et de six cages pour les animaux témoins ne recevant que la solution aqueuse de gomme arabique par voie intrapéritonéale. Une demi-heure plus tard, les deux groupes d'une même cage sont réunis et on note le nombre de combats qui surviennent en 10 minutes. Les résultats sont consignés dans le tableau I qui suit et démontrent que le CRL 40 636 selon l'invention (i) diminue fortement le nombre de combats à la dose de 64 mg/kg, (ii) supprime totalement les combats à la dose de 128 mg/kg, et (iii) possède un effet anti-agressif bénéfique nettement supérieur à celui du CRL 40 473 selon l'exemple 18 du brevet belge précité, d'une part, et à celui du CRL 40 816, d'autre part.

TABLEAU I

Essais comparatifs

| Produit | No. de code | Dose (mg/kg) | Nombre de combats par souris | Diminution du nombre de combats par rapport aux témoins |
|---|---|---|---|---|
| témoins | — | — | 3,05 | 0% |
| Ex 1 | CRL 40636 | 64 | 1,48 | 51% |
| Ex 1 | CRL 40636 | 128 | 0 | 100% |
| CP-1 | CRL 40473 | 128 | 1,80 | 40% |
| CP-1 | CRL 40473 | 512 | 2,23 | 26% |
| CP-2 | CRL 40816 | 128 | 1,42 | 53% |
| CP-2 | CRL 40816 | 512 | 0 | 100% |

Note: formules développées des produits

Ex 1 :

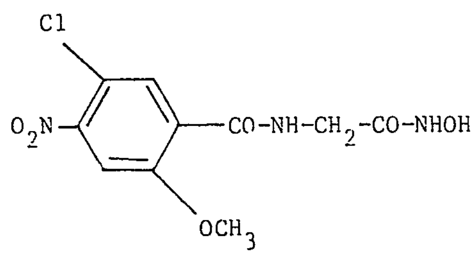

CP-1 :

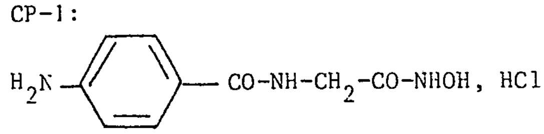

CP-2 :

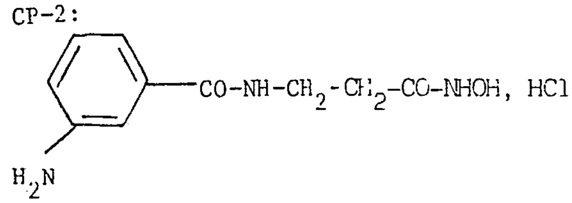

*X. ACTION VIS-A-VIS DE QUELQUES COMPORTEMENTS PERTURBES PAR DIVERS AGENTS*

*1°) Motilité réduite par habituation à l'enceinte*

Après 18 heures de séjour dans les actimètres, les souris (6 par dose, 12 témoins) reçoivent le CRL 40 636. Elles sont aussitôt replacées dans leurs enceintes respectives et, une demi-heure plus tard, on enregistre leur motilité pendant 30 minutes.

On observe que le CRL 40 636 n'entraîne pas une reprise nette de l'activité motrice chez la souris habituée à son enceinte.

*2°) Motilité réduite par agression hypoxique*

Une demi-heure après avoir reçu le CRL 40 636, les souris (10 par dose, 20 témoins) sont soumises à une anoxie hypobare aiguë [dépression de 600 mmHg (i.e. environ 8 x $10^4$ Pa) en 90 secondes; détente de 45 secondes], puis elles sont placées en actimètre où leur motilité est enregistrée pendant 10 minutes.

On observe que le CRL 40 636 ne provoque pas d'amélioration nette de la récupération motrice chez la souris dont la motilité a été déprimée à la suite d'un séjour bref dans une enceinte à pression réduite.

*3°) Anoxie asphyxique*

Des lots de 10 souris reçoivent le CRL 40 636 une demi-heure avant l'administration intrapéritonéale de 32 mg/kg de triiodoéthylate de gallamine (agent curarisant de référence).

On observe que le CRL 40 636 ne modifie pas le délai d'apparition des convulsions et de la mort consécutives à une anoxie asphyxique provoquée par un curarisant.

*XI. ESSAI COMPLEMENTAIRE*

Un essai complémentaire a été réalisé avec le produit selon l'invention, celui-ci étant administré en suspension dans une solution aqueuse de gomme arabique, par voie gastrique, sous un volume de 5 ml/kg, à des lots de 6 rats mâles 0,5 h ou 1 h avant l'administration de 2 mg/kg intrapéritonéale d'amphétamine.

On constate que le CRL 40 636 antagonise brièvement les stéréotypies induites par l'amphétamine chez le rat.

*XII. CONCLUSIONS*

Les résultats des essais donnés ci-dessus mettent en évidence que le CRL 40 636 présente dans son profil neuropsychopharmacologique des effets essentiellement sédatifs très intenses, objectivés par une hypomotilité, une hypothermie, une diminution des réactivités, et une diminution de l'agressivité intergroupes, et qu'il est pratiquement dépourvu d'effets antidépresseurs (pas d'antagonisme des hypothermies induites par l'apomorphine, la réserpine et l'oxotrémorine).

En clinique le CRL 40 636 s'est révélé chez l'homme adulte comme étant un excellent sédatif par voie orale à la dose quotidienne de 50 à 100 mg. La posologie à utiliser consiste notamment à administrer deux à quatre comprisés ou gélules par jour renfermant chacun 25 mg de CRL 40 636.

On préconise l'utilisation de l'acide 2-(5-chloro-4-nitro-2-méthoxybenzamido)-acétohydroxamique pour l'obtention d'un médicament sédatif destiné à un usage en thérapeutique chez les patients ayant besoin d'un tel médicament, notamment dans les cas de surexcitation.

**Revendications pour les états contractants:**
BE, CH, DE, FR, GB, IT, LI, LU, NL, SE

1. Acide 2-(5-chloro-4-nitro-2-méthoxybenzamido)-acétohydroxamique.

2. Composition thérapeutique, caractérisée en ce qu'elle renferme, en association avec un excipient physiologiquement acceptable, l'acide 2-(5-chloro-4-nitro-2-méthoxybenzamido)-acétohydroxamique en tant que principe actif.

3. Procédé de préparation de l'acide 2-(5-chloro-4-nitro-2-méthoxybenzamido)-acétohydroxamique, caractérisé en ce que l'on fait réagir l'hydroxylamine avec un 5-chloro-4-nitro-2-méthoxybenzamidoacétate d'alkyle inférieur en $C_1$-$C_3$.

4. Utilisation de l'acide 2-(5-chloro-4-nitro-2-méthoxybenzamido)-acétohydroxamique pour l'obtention d'un médicament sédatif destiné à un usage en thérapeutique chez les patients ayant besoin d'un tel médicament.

**Revendication pour l'état contractant:** AT

1. Procédé de préparation de l'acide 2-(5-chloro-4-nitro-2-méthoxybenzamido)-acétohydroxamique, caractérisé en ce que l'on fait réagir l'hydroxylamine avec un 5-chloro-4-nitro-2-méthoxybenzamidoacétate d'alkyle inférieur en $C_1$-$C_3$.

**Patentansprüche für Vertragsstaaten:**
BE, CH, FR, GB, IT, LI, LU, NL, SE

1. 2-(5-Chlor-4-nitro-2-methoxybenzamido)-acetohydroxamsäure.

2. Therapeutisches Mittel, dadurch gekennzeichnet, daß es zusammen mit einem physiologisch zulässigen Bindemittel 2-(5-Chlor-4-nitro-2-methoxybenzamido)-acetohydroxamsäure als Wirkstoff enthält.

3. Verfahren zur Herstellung von 2-(5-Chlor-4-nitro-2-methoxybenzamido)-acetohydroxamsäure, dadurch gekennzeichnet, daß man Hydroxylamin mit einem Niederalkyl $C_1$-$C_3$-5-chlor-4-nitro-2-methoxybenzamido-acetat umsetzt.

4. Verwendung von 2-(5-Chlor-4-nitro-2-methoxybenzamido)-acetohydroxamsäure zur Herstellung eines sedativen Medikamentes, das zur therapeutischen Anwendung bei Patienten, die ein derartiges Medikament benötigen, bestimmt ist.

**Patentanspruch für Vertragsstaat:** AT

Verfahren zur Herstellung von 2-(5-chlor-4-nitro-2-methoxybenzamido)-acetohydroxamsäure, dadurch gekennzeichnet, daß man Hydroxylamin mit

einem Niederalkyl $C_1$-$C_3$-5-Chlor-4-nitro-2-methoxybenzamidoacetat umsetzt.

**Claims for the contracting states:**
BE, CH, DE, FR, GB, IT, LI, LU, NL, SE

1. 2-(5-Chloro-4-nitro-2-methoxybenzamido)-acetohydroxamic acid.

2. A therapeutic composition comprising, in association with a physiologically acceptable excipient, a pharmaceutically effective amount of 2-(5-chloro-4-nitro-2-methoxybenzamido)acetohydroxamic acid according to claim 1.

3. A method for preparing the compound according to claim 1, which comprises reacting hydroxylamine with a $C_1$-$C_3$ lower alkyl 5-chloro-4-nitro-2--methoxybenzamidoacetate.

4. A use of 2-(5-chloro-4-nitro-2-methoxybenzamido)acetohydroxamic acid for abtaining a sedative medicament destined to a therapeutical use for patient in need of such a medicament.

**Claim for the contracting state:** AT

1. A method for preparing the compound according to claim 1, which comprises reacting hydroxylamine with a $C_1$-$C_3$ lower alkyl 5-chloro-4-nitro-2--methoxybenzamidoacetate.